# EUROPEAN PATENT APPLICATION

(11) **EP 3 865 108 A1**
(43) Date of publication of application: **18.08.2021**
(21) Application number: 19870659.0
(22) Date of filing: 11.10.2019
(51) Int. Cl.: A61H 15/00, A61B 5/00

(54) **MASSAGE DEVICE COMPRISING BODY COMPOSITION MEASUREMENT MODULE FOR BODY COMPOSITION MEASUREMENT, AND METHOD OF CONTROLLING SAME**

(30) Priority: 12.10.2018 KR 20180121988
(71) Applicant: Bodyfriend Co., Ltd., Seoul 06302 (KR)
(72) Inventor: KIM, Do Hyun, Seongnam-si Gyeonggi-do 13368 (KR); KIM, Jin Hwan, Gunpo-si Gyeonggi-do 15886 (KR); KANG, Woong Chul, Seoul 06302 (KR); CHO, Soo Hyun, Seoul 06302 (KR); JEON, Chul Jin, Seoul 06302 (KR); KONG, Deok Hyun, Seoul 06302 (KR)
(74) Representative: Frenkel, Matthias Alexander
(86) International application number: PCT/KR2019/013339
(87) International publication number: WO 2020/076115

(57) **Abstract**

Disclosed herein are a massage device including a body composition measurement module for body composition measurement and a method of controlling the same. An embodiment of the present disclosure discloses a massage device including a massage module configured to provide mechanical stimuli to at least a part of the body of a user, a first body composition measurement module provided on a leg massage part to measure the body composition of the user, and a controller configured to control operation of the massage module and the first body composition measurement module.

## Description

### [Technical Field]

The present disclosure relates to a massage device capable of measuring body composition and a method of controlling the same.

### [Background Art]

A massage is an adjuvant therapy in which mechanical stimuli in various forms are applied to a part of a subject's body by rubbing, pressing, pulling, tapping, or moving the part of the body to adjust modulation of the subject's body, aid circulation, and relieve the subject's fatigue.

For economic and time reasons, an increase in demand for massages has caused an increase in demand for massage devices or massage apparatuses that provide artificial massage functions. That is, with an increase in demand to relieve fatigue or stress by relaxing tight muscles through a massage, various massage devices which are efficient in terms of time and cost have been launched. Tools, devices, or apparatuses in any form that perform a massage through mechanical devices without a massager are referred to as massage devices.

Further, there has been an increase in user demand for massage devices that not only relieve fatigue or stress but also promote health through a massage.

Therefore, research for providing a massage device capable of promoting user health has been continuously carried out.

### [Disclosure]

### [Technical Problem]

One objective of the present disclosure is to provide a massage device capable of measuring the body composition of a user and a method of controlling the same.

### [Technical Solution]

A first aspect of the present disclosure provides a massage device including a massage module configured to provide mechanical stimuli to at least a part of the body of a user, a first body composition measurement module provided on a leg massage part to measure the body composition of the user, and a controller configured to control the operation of the massage module and the first body composition measurement module.

Also, the first body composition measurement module may include an upper housing which includes an empty space formed therein and at least one opening formed in an upper portion thereof, a first electrode part which has at least a portion inserted into the upper housing and which protrudes to the outside of the upper housing through the at least one opening, an elastic part configured to allow the first electrode part and a lower housing to be elastically coupled to each other, and the lower housing coupled to the upper housing.

Also, a protruding part may be provided on a lower portion of the first electrode part, the protruding part may pass through a hole provided in the lower housing and protrude to the outside of the first body composition measurement module, and information on a user may be transmitted to the massage device through the protruding part.

Also, the massage device may further include an arm massage part configured to massage an arm of the user and a second body composition measurement module provided on the arm massage part, the arm massage part may include an arm massage part frame constituting a framework of the arm massage part and a hand acupressure part configured to apply acupressure to a hand of the user, and the second body composition measurement module may be provided on the hand acupressure part.

Also, in a case of performing body composition measurement, the controller may adjust a posture of the massage device and then perform the body composition measurement.

Also, the massage device may lift the leg massage part and then perform the body composition measurement.

Also, the massage device may recline a body massage part and then perform the body composition measurement.

Also, the massage device may lift the leg massage part, recline the body massage part, and then perform the body composition measurement.

A second aspect of the present disclosure provides a method of measuring body composition using a massage device including a body massage part and a leg massage part, the method including receiving an input relating to body composition measurement, lifting the leg massage part, and, after the leg massage part is lifted, performing the body composition measurement.

A third aspect of the present disclosure provides a method of measuring body composition using a massage device including a body massage part and a leg massage part, the method including receiving an input relating to body composition measurement, reclining the body massage part, and, after the body massage part is reclined, performing the body composition measurement.

Also, the method according to the second aspect may further include reclining the body massage part, and in the performing of the body composition measurement, body composition may be measured after the leg massage part is lifted and the body massage part is reclined.

### [Advantageous Effects]

A massage device according to an embodiment of the present disclosure can measure the body composition of a user through a body composition measurement module. Also, the body composition of the user that is acquired through the body composition measurement module can be utilized to provide a massage that is customized to the user.

Advantageous effects of the present disclosure are not limited to those mentioned above, and other unmentioned advantageous effects should be clearly understood by those of ordinary skill in the art to which the present disclosure pertains from the description below.

### [Description of Drawings]

Various aspects will be described below with reference to the drawings. Here, similar reference numerals will be used to refer to substantially similar elements. In the following embodiments, for the sake of description, a plurality of specific details will be proposed to provide overall understanding of one or more aspects. However, it is apparent that the aspect(s) may be embodied without the specific details. In other examples, known structures and devices are illustrated as block diagrams to facilitate description of one or more aspects.
FIG. 1 is a view for describing a massage device 1000 according to an embodiment of the present disclosure.
FIG. 2 is a view for describing a main frame according to an embodiment of the present disclosure.
FIG. 3 is a view for describing a first body composition measurement module 310 according to an embodiment of the present disclosure.
FIG. 4 is a view for describing a method in which a user measures his or her body composition through the first body composition measurement module 310 according to an embodiment of the present disclosure.
FIG. 5 is a view for describing a structure of the first body composition measurement module 310 according to an embodiment of the present disclosure.
FIGS. 6 and 7 are views for describing a second body composition measurement module 1940 according to an embodiment of the present disclosure.
FIG. 8 is a view for describing elements of the massage device 1000 according to an embodiment of the present disclosure.
FIG. 9 is a view for describing an embodiment in which a posture of the massage device 1000 is adjusted for body composition measurement according to an embodiment of the present disclosure.
FIG. 10 is a view for describing a concept of a method of utilizing the first body composition measurement module 310 and the second body composition measurement module 1940 to acquire an electrocardiograph.

### [Best Mode of the Invention]

A massage device including a massage module configured to provide mechanical stimulation to at least a part of the body of a user, a first body composition measurement module provided on a leg massage part to measure the body composition of the user, and a controller configured to control the operation of the massage module and the first body composition measurement module.

### [Modes of the Invention]

The objects, features, and advantages of the present disclosure described above will become more apparent through the following embodiments relating to the accompanying drawings. The following descriptions of specific structures or functions are only given to describe embodiments according to the concept of the present disclosure. The embodiments according to the concept of the present disclosure may be embodied in various forms, and the present disclosure should not be interpreted as being limited by the embodiments described in the present specification or application.

Since the embodiments according to the concept of the present disclosure may be changed in various ways and have various forms, specific embodiments are illustrated in the drawings and will be described in detail in the present specification or application. However, this does not limit the embodiments according to the concept of the present disclosure to specific disclosed forms, and all changes, equivalents, and substitutes included in the idea and technical scope of the present disclosure should be construed as belonging to the embodiments according to the concept of the present disclosure.

Terms such as first and/or second may be used to describe various elements, but the elements are not limited by the terms. The terms are only used for the purpose of distinguishing one element from another element. For example, without departing from the scope according to the concept of the present disclosure, a first element may be referred to as a second element and, likewise, a second element may also be referred to as a first element.

When it is mentioned that a certain element is "connected" or "linked" to another element, although the certain element may be directly connected or linked to the other element, it should be understood that another element may be present therebetween. On the other hand, when it is mentioned that a certain element is "directly connected" or "directly linked" to another element, it should be understood that another element is not present therebetween. Other expressions used to describe a relationship between elements, i.e., "between" and "directly between" or "adjacent" and "directly adjacent," should be interpreted likewise.

Terms used in the present specification are only used to describe specific embodiments and are not intended to limit the present disclosure. A singular expression includes a plural expression unless the context clearly indicates otherwise. In the specification, terms such as "include" or "have" should be understood as designating that features, number, steps, operations, elements, parts, or combinations thereof are present and not as precluding the presence of or the possibility of adding one or more other features, numbers, steps, operations, elements, parts, or combinations thereof in advance.

Unless otherwise defined, all terms including technical or scientific terms used herein have the same meaning as commonly understood by those of ordinary skill in the art to which the present disclosure pertains. Terms, such as those defined in commonly used dictionaries, should be construed as having a meaning that is consistent with their meaning in the context of the relevant art and are not to be construed in an idealized or overly formal sense unless expressly so defined herein.

In the present specification, an actuator refers to an element capable of providing a driving force. Examples of the actuator may include a motor, a linear motor, an electronic motor, a DC motor, an AC motor, a linear actuator, an electric actuator, and the like, but are not limited thereto.

In the present specification, a spiral rod refers to a linear member having a spiral groove and may be implemented with a metal material. Examples of the spiral rod may include a cylindrical bar having a spiral groove formed in a surface thereof. Examples of the spiral rod may also include a metal lead screw.

In the present specification, body composition refers to information on components constituting the body. For example, body composition may include information on intracellular water, extracellular water, body water, proteins, minerals, body fat, muscle mass, fat mass, skeletal muscle mass, body fat percentage, body mass index (BMI), muscle mass for each body part, body water for each body part, generalized edema, edema for each body part, somatic cell mass, bone mineral content, waist-hip ratio, visceral fat area, basal metabolic rate, and the like, but is not limited thereto.

In a case in which a massage device 1000 causes weak AC to flow in the human body through a body composition measurement module, the current flows along body water which is highly conductive, and the amount of body water determines the width of a path through which electricity flows. This is represented by a measured value referred to as impedance, and the massage device may utilize the measured impedance to calculate body composition. In this case, the massage device 1000 may calculate body composition using the 2-electrode, 4-point touch electrode method using two electrodes or may calculate body composition using the 4-electrode, 8-point touch electrode method using four electrodes.

In the present specification, a binaural beat may refer to a specific form of audio information that may regulate brain waves.

In the present specification, according to an embodiment, a massage device may refer to a massage device including a body massage part and a leg massage part. Also, according to another embodiment, a body massage part 100 and a leg massage part 300 may be present as separate devices (for example, a body massage device and a leg massage device), and a massage device may refer to the body massage device or the leg massage device.

Hereinafter, embodiments of the present disclosure will be described in more detail with reference to the accompanying drawings.

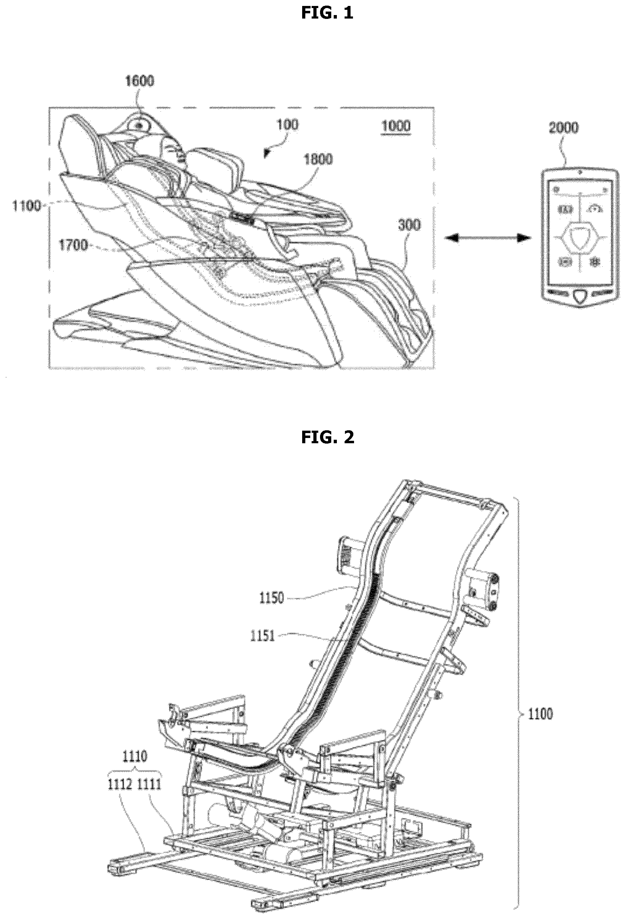

FIG. 1 is a view for describing a massage device 1000 according to an embodiment of the present disclosure.

The massage device 1000 according to an embodiment of the present disclosure may include a body massage part 100 that has an area formed to accommodate at least a portion of a user's body and that is configured to massage the user's torso and a leg massage part 300 configured to massage the user's legs.

The body massage part 100 may provide a massage to at least a portion of the user's body. The body massage part 100 may include a massage module 1700 configured to provide a massage function to at least a portion of the user's body, an audio output module 1600 configured to provide an audio output in an arbitrary form to the user, a main frame 1100 constituting a framework of the body massage part 100, and a user input part 1800 configured to receive an input in an arbitrary form from the user.

The above-described elements that the body massage part 100 includes are merely an exemplary embodiment, and the body massage part 100 may include various elements other than those described above.

Also, the shape and structure of the massage device 1000 illustrated in FIG. 1 are merely illustrative, and a massage device 1000 having various other forms may also fall within the scope of the present disclosure unless the form of the massage device 1000 deviates from the scope defined by the claims of the present disclosure.

The body massage part 100 may have a space formed in an arbitrary shape to accommodate a user. The body massage part 100 may have a space formed in a shape that corresponds to a shape of the user's body. For example, as illustrated in FIG. 1, the body massage part 100 may be implemented as a seating type and may accommodate the entire body of the user or a portion of the body.

A portion of the body massage part 100 that comes in contact with the ground may include an arbitrary material configured to increase a frictional force or an arbitrary member configured to increase a frictional force (e.g., a nonslip pad etc.) and may include a wheel configured to reinforce the mobility of the massage device 1000.

At least a portion of the body massage part 100 may be able to slide. For example, in the case in which the body massage part 100 begins to perform a massage, at least a portion of the body massage part 100 may slide forward. Also, the body massage part 100 may be reclined. As a result, the body massage part 100 may provide a massage while reclined.

According to an embodiment of the present disclosure, the massage device 1000 may include at least one air cell (not illustrated). The air cell may be located at portions of the massage device 1000 that correspond to the user's shoulders and pelvis, arm massage parts, the leg massage part 300, and the like, but the present disclosure is not limited thereto, and the air cell may be disposed at various other portions of the massage device 1000.

The massage device 1000 may include an air supply part. The air supply part may supply air to the air cell to inflate the air cell. The air supply part may be located inside the body massage part 100 or located on the leg massage part 300. Also, the air supply part may be located outside the massage device 1000.

The leg massage part 300 may provide a leg massage to the user. For example, the leg massage part 300 may include a calf massage part configured to massage a user's calf and/or a foot massage part configured to massage a user's foot.

A length of the leg massage part 300 may be adjustable according to characteristics of the user's body. For example, in a case in which a tall user uses the massage device 1000, since the length of the user's calf is long, it is necessary to increase the length of the leg massage part 300. Also, in a case in which a short user uses the massage device 1000, since the length of the user's calf is short, it is necessary to decrease the length of the leg massage part 300. Accordingly, the leg massage part 300 may provide a leg massage that is customized to a height of the user.

The massage module 1700 may be provided inside the body massage part 100 so as to provide mechanical stimuli in arbitrary forms to a user accommodated in the body massage part 100. As illustrated in FIG. 1, the massage module 1700 may move along the main frame 1100 provided inside the body massage part 100.

For example, a rack gear may be provided on the main frame 1100 of the body massage part 100, and the massage module 1700 may, while moving along the rack gear, provide mechanical stimuli to various parts of the user's body. The massage module 1700 may include a ball massage unit or a roller massage unit, but the present disclosure is not limited thereto.

The main frame 1100 constitutes a framework of an internal configuration of the body massage part 100 and may be implemented with a metal material, a plastic material, or the like. For example, the main frame 1100 may be implemented with iron, alloys, steel, and the like, but the present disclosure is not limited thereto, and the main frame 1100 may also be implemented with various other rigid materials.

According to an embodiment of the present disclosure, the massage device 1000 may include the audio output module 1600. The audio output module 1600 may be provided at various locations. For example, the audio output module 1600 may include a plurality of output units such as an upper-end audio output unit disposed on an upper end of a seat part coming in contact with the user, a front audio output unit attached to front ends of the arm massage parts at the left and right sides of the seat part, and/or a rear audio output unit attached to rear ends of the arm massage parts, but the present disclosure is not limited thereto. In this case, the audio output module 1600 may provide stereophonic sound such as 5.1 surround sound, but the present disclosure is not limited thereto.

According to an embodiment of the present disclosure, the user may control the massage device 1000 using a massage device control device 2000. The massage device control device 2000 may be connected to the massage device 1000 through wired communication and/or wireless communication.

The massage device control device 2000 may include a remote controller, a cellular phone, a personal digital assistant (PDA), and the like, but the present disclosure is not limited thereto, and the massage device control device 2000 may include various other electronic devices that may be connected to the massage device 1000 through wired or wireless communication.

FIG. 2 is a view for describing the main frame according to an embodiment of the present disclosure.

According to an embodiment of the present disclosure, the main frame 1100 may include an upper frame 1150 on which the massage module 1700 is provided and a base frame 1110 configured to support the upper frame 1150.

A rack gear 1151 may be provided on at least a portion of the upper frame 1150. The rack gear 1151 is a member configured to guide the vertical movement of the massage module 1700 and may include a plurality of valley portions and a plurality of ridge portions.

According to an embodiment of the present disclosure, the rack gear 1151 may be provided to face both side portions of the upper frame 1150, and the massage module 1700 may move along the rack gear 1151.

For example, the massage module 1700 may include a gear engaged with the rack gear 1151, and as the gear rotates due to an actuator provided in the massage module 1700, the massage module 1700 may move upward or downward.

The rack gear 1151 may be implemented with a metal material or a plastic material. For example, the rack gear 1151 may be implemented with iron, steel, alloys, reinforced plastic, a melamine resin, a phenol resin, and the like, but the present disclosure is not limited thereto.

The upper frame 1150 may be implemented in various shapes. For example, the upper frame 1150 may be classified into an S-frame, an L-frame, an S&L frame, or a double S&L frame according to its shape, but the present disclosure is not limited thereto.

The S-frame refers to the upper frame 1150 in which at least a portion is curved in an S-like shape. The L-frame refers to the upper frame 1150 in which at least a portion is bent in an L-like shape, the S&L frame refers to a frame that includes both a portion curved in an S-like shape and a portion bent in an L-like shape, and the double S&L frame refers to a frame that includes a portion bent in an L-like shape and two portions curved in an S-like shape.

The base frame 1110 refers to a portion of the main frame 1100 that supports the upper frame 1150 and comes in contact with the ground. The base frame 1110 may include a base upper frame 1111 and a base lower frame 1112.

The base upper frame 1111 may support the upper frame 1150, and the base lower frame 1112 may come in contact with the ground. Also, the base upper frame 1111 may be located so as to come in contact with the base lower frame 1112.

According to an embodiment of the present disclosure, the base upper frame 1111 may move along the base lower frame 1112. For example, the base upper frame 1111 may slide forward or rearward along the base lower frame 1112. In this case, the upper frame 1150 may be connected to the base upper frame 1111 and move according to the movement of the base upper frame 1111.

For example, in the case in which the base upper frame 1111 moves forward, the upper frame 1150 may also move forward, and in the case in which the base upper frame 1111 moves rearward, the upper frame 1150 may also move rearward. Thus, sliding of the body massage part 100 may be allowed.

Specifically, in order to allow the movement of the base upper frame 1111, a moving wheel may be provided on a lower portion of the base upper frame 1111. Also, a guide member configured to guide the moving wheel may be provided at an upper portion of the base lower frame 1112. The moving wheel provided on the base upper frame 1111 may move along the guide member provided on the base lower frame 1112 so that forward movement or rearward movement of the base upper frame 1111 is allowed.

According to another embodiment of the present disclosure, the massage device 1000 may not provide a sliding function and, in this case, the base frame 1110 may not be separated into upper and lower frames.

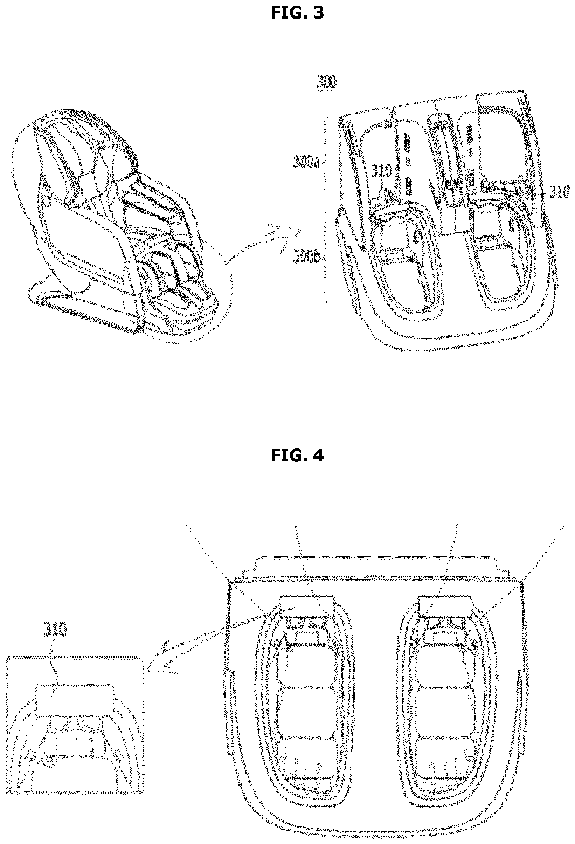

FIG. 3 is a view for describing a first body composition measurement module 310 according to an embodiment of the present disclosure, and FIG. 4 is a view for describing a method in which a user measures his or her body composition through the first body composition measurement module 310 according to an embodiment of the present disclosure.

According to an embodiment of the present disclosure, the massage device 1000 may include at least one of the first body composition measurement module 310 and a second body composition measurement module 1940. The first body composition measurement module 310 may be provided on the leg massage part 300, and the second body composition measurement module 1940 may be provided on at least one of both arm massage parts 1900. The massage device 1000 may utilize the first body composition measurement module 310 or the second body composition measurement module 1940 to measure body composition using a 2-pole electrode method or may utilize both the first body composition measurement module 310 and the second body composition measurement module 1940 to measure body composition using a 4-pole electrode method.

Also, according to another embodiment of the present disclosure, the massage device 1000 may utilize each of the first body composition measurement module 310 and the second body composition measurement module 1940 to measure body composition using the 2-pole electrode method and may select a highly reliable measurement result through a reliability test to acquire the body composition of a user.

The first body composition measurement module 310 may be disposed on at least a portion of the leg massage part 300 and, in a case in which a user is seated on the massage device 1000, may come in contact with at least a portion of a leg of the user.

For example, the leg massage part 300 may be divided into a calf massage part 300a configured to massage a user's calf and a foot massage part 300b configured to massage a user's foot, and the first body composition measurement module 310 may be located between the calf massage part 300a and the foot massage part 300b.

Also, the first body composition measurement module 310 may be provided on at least a portion of the foot massage part 300b or located on at least a portion of the calf massage part 300a.

In the case in which the user is seated on the massage device 1000, the first body composition measurement module 310 may come in contact with a portion between the user's calf and foot.

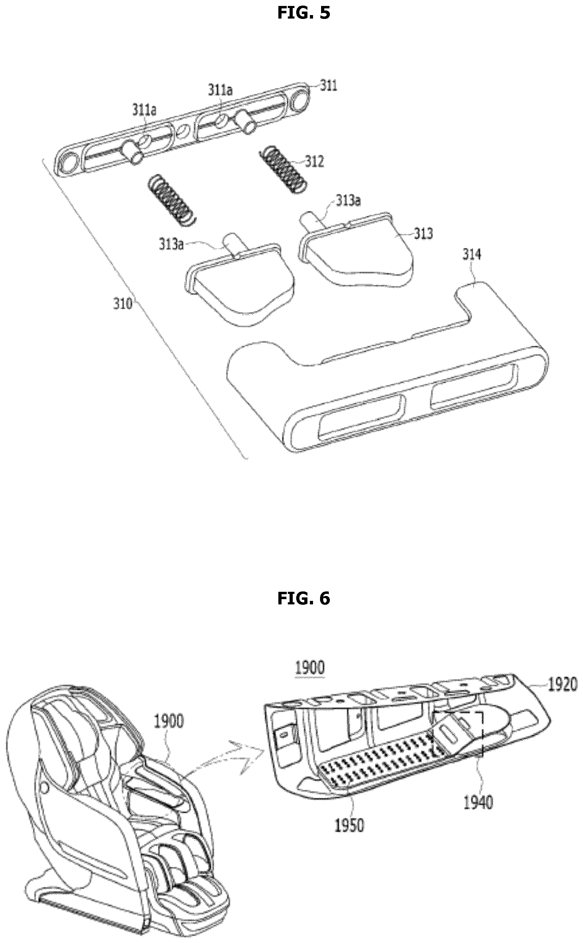

FIG. 5 is a view for describing a structure of the first body composition measurement module 310 according to an embodiment of the present disclosure.

According to an embodiment of the present disclosure, the first body composition measurement module 310 may include at least one of an upper housing 314, a lower housing 311, a first electrode part 313, and an elastic part 312.

The upper housing 314 includes an empty space formed therein, and at least a portion of the first electrode part 313 may be inserted into the upper housing 314. Also, the upper housing 314 includes at least one opening formed in an upper portion thereof, and the first electrode part 313 may protrude to the outside of the upper housing 314 through the at least one opening.

The first electrode part 313 may be elastically coupled to the lower housing 311 through the elastic part 312. Also, a protruding part 313a may be provided on a lower portion of the first electrode part 313, the protruding part 313a may pass through a hole 311a provided in the lower housing 311 and protrude to the outside of the first body composition measurement module 310, and information on a user may be transmitted to the massage device 1000 through the protruding part 313a.

According to an embodiment of the present disclosure, in a case in which at least a portion of a leg of the user comes in contact with the first body composition measurement module 310, pressure may be applied to the first body composition measurement module 310 by the user, and the elastic part 312 may absorb the pressure. As a result, the first electrode part 313 may come in close contact with the user's body, and thus the efficiency of body composition measurement may be increased.

The upper housing 314 and the lower housing 311 may be implemented with plastic or reinforced plastic, but the present disclosure is not limited thereto. Also, the first electrode part 313 may be implemented with a conductive metal material, but the present disclosure is not limited thereto. The elastic part 312 is illustrated as a spring for convenience of description, but the present disclosure is not limited thereto, and the elastic part 312 may be implemented with various materials that provide elasticity.

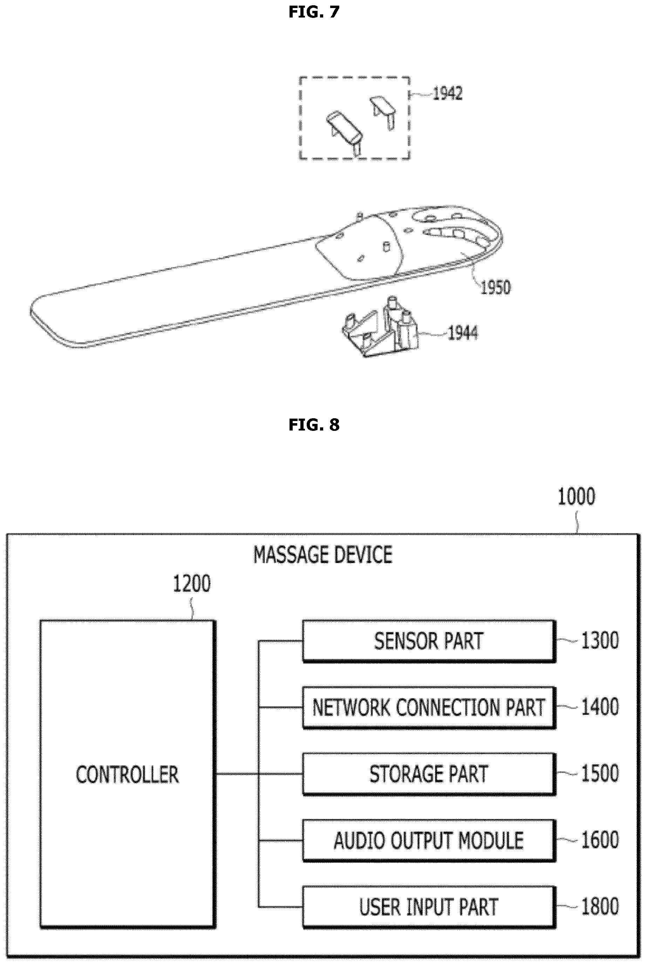

FIGS. 6 and 7 are views for describing the second body composition measurement module 1940 according to an embodiment of the present disclosure.

According to an embodiment of the present disclosure, the second body composition measurement module 1940 may be provided on the arm massage part 1900. For example, the second body composition measurement module 1940 may be provided on one arm massage part 1900 or provided on each of the arm massage parts 1900 at both sides.

An arm massage part frame 1920 constituting a framework of the arm massage part 1900 may be provided inside the arm massage part 1900, a hand acupressure part 1950 may be provided on the arm massage part frame 1920, and the second body composition measurement module 1940 may be provided on the hand acupressure part 1950.

The arm massage part frame 1920 constitutes a framework of an internal configuration of the arm massage part 1900 and may be implemented with a metal material, a plastic material, or the like. For example, the arm massage part frame 1920 may be implemented with iron, alloys, steel, and the like, but the present disclosure is not limited thereto, and the arm massage part frame 1920 may be implemented with various other rigid materials.

The hand acupressure part 1950 may be installed on the arm massage part 1900 of the massage device 1000 to press different portions of a user's palm by pressure applied thereto from above. The hand acupressure part 1950 has a plurality of protrusions arranged on a surface of a spherical, elliptical, or dome-shaped body to apply acupressure to arbitrary portions of fingers and a palm, and since a plurality of magnets may be provided on the dome-shaped body, blood circulation of the user may be enhanced.

The hand acupressure part 1950 may be implemented with a metal material, a plastic material, or the like. For example, the hand acupressure part 1950 may be implemented with iron, alloys, steel, plastic, reinforced plastic, and the like, but the present disclosure is not limited thereto, and the hand acupressure part 1950 may be implemented with various other rigid materials.

The second body composition measurement module 1940 may include a second electrode part 1942. Also, the second body composition measurement module 1940 may include a second body composition measurement support part 1944 configured to couple the second electrode part 1942 to the hand acupressure part 1950.

A plurality of openings may be provided in the hand acupressure part 1950, and at least a portion of the second electrode part 1942 may be coupled to the second body composition measurement support part 1944 through the openings provided in the hand acupressure part 1950.

FIG. 8 is a view for describing elements of the massage device 1000 according to an embodiment of the present disclosure.

According to an embodiment of the present disclosure, the massage device 1000 may include at least one of a controller 1200, a sensor part 1300, the user input part 1800, the audio output module 1600, and a network connection part 1400.

The controller 1200 may control the operation of the massage device 1000. The controller 1200 may be implemented with a single processor or implemented with a plurality of processors. In a case in which the controller 1200 is implemented with a plurality of processors, at least some of the plurality of processors may be located to be physically spaced apart at a certain distance. The controller 1200 is not limited thereto and may be implemented in various other ways.

According to an embodiment of the present disclosure, the controller 1200 may control the operation of the massage device 1000. For example, the massage device 1000 may include a plurality of actuators, and the controller 1200 may control the operation of the plurality of actuators to control the operation of the massage device 1000. For example, the massage device 1000 may include a massage module 1700 moving actuator, at least one actuator included in the massage module, and at least one of a back angle actuator, a leg angle actuator, a foot massage actuator, a leg length adjusting actuator, and a sliding actuator, and the controller 1200 may control the actuators to control the operation of the massage device 1000.

The massage module moving actuator is an actuator that allows vertical movement of the massage module 1700, and the massage module 1700 may move along the rack gear due to the operation of the massage module 1700 moving actuator.

The back angle actuator is an actuator that adjusts an angle of a portion of the massage device 1000 that comes in contact with the user's back, and the back angle of the massage device 1000 may be adjusted due to the operation of the back angle actuator.

The leg angle actuator is an actuator that adjusts an angle of the leg massage part 300 of the massage device 1000, and an angle between the leg massage part 300 and the body massage part 100 may be adjusted due to the operation of the leg angle actuator.

The foot massage actuator refers to an actuator that operates a foot massage module included in the leg massage part 300. The massage device 1000 may utilize the foot massage actuator to provide a foot massage to the user.

At least one actuator may be included in the massage module 1700, and the controller 1200 may operate the at least one actuator to provide various massage operations. For example, the controller 1200 may operate at least one actuator included in the massage module 1700 to provide a tapping massage, a rubbing massage, and the like, but the present disclosure is not limited thereto, and the controller 1200 may provide various other massage operations.

The leg length adjusting actuator refers to an actuator that adjusts the length of the leg massage part 300. For example, the controller 1200 may utilize the leg length adjusting actuator to adjust the length of the leg massage part 300 to suit each user, and as a result, a user may receive a massage that is suitable for his or her body frame.

The sliding actuator allows sliding of the massage device 1000. For example, a horizontal base upper frame 1114a may move forward or rearward due to the operation of the sliding actuator, and as a result, an upper frame connected to the horizontal base upper frame 1114a may also move forward or rearward.

According to an embodiment of the present disclosure, the controller 1200 may control the operation of the first body composition measurement module 310 and/or the second body composition measurement module 1940.

For example, the controller 1200 may determine whether at least a portion of a user's leg is in contact with the first body composition measurement module 310, may determine whether at least a portion of a user's hand is in contact with the second body composition measurement module 1940, and may, on the basis of determined results relating to contact, control the operation of the first body composition measurement module 310 and/or the second body composition measurement module 1940.

Specifically, in a case in which at least a portion of a user's leg is in contact with the first body composition measurement module 310, the controller 1200 may perform body composition measurement using the 2-electrode, 4-point touch electrode method by utilizing the first body composition measurement module 310, and in a case in which at least a portion of a user's hand is in contact with the second body composition measurement module 1940, the controller 1200 may perform body composition measurement using the 2-electrode, 4-point touch electrode method by utilizing the second body composition measurement module 1940. Also, in a case in which at least a portion of the user's leg is in contact with the first body composition measurement module 310 and at least a portion of the user's hand is in contact with the second body composition measurement module 1940, the controller 1200 may perform body composition measurement using the 4-electrode, 8-point touch electrode method.

As another example, the controller 1200 may, through the sensor part 1300, determine whether the legs of a user are placed in the leg massage part 300 and determine whether an arm of the user is placed in at least one of the arm massage parts 1900 and may, on the basis of determined results relating to placement, control the operation of the first body composition measurement module 310 and/or the second body composition measurement module 1940.

Specifically, in a case in which the legs of a user are placed in the leg massage part 300, the controller 1200 may perform body composition measurement using the 2-electrode, 4-point touch electrode method by utilizing the first body composition measurement module 310, and in a case in which an arm of the user is placed in the arm massage part 1900, the controller 1200 may perform body composition measurement using the 2-electrode, 4-point touch electrode method by utilizing the second body composition measurement module 1940. Also, in a case in which the user's legs are placed in the leg massage part 300 and the user's arm is placed in the arm massage part, the controller 1200 may perform body composition measurement using the 4-electrode, 8-point touch electrode method.

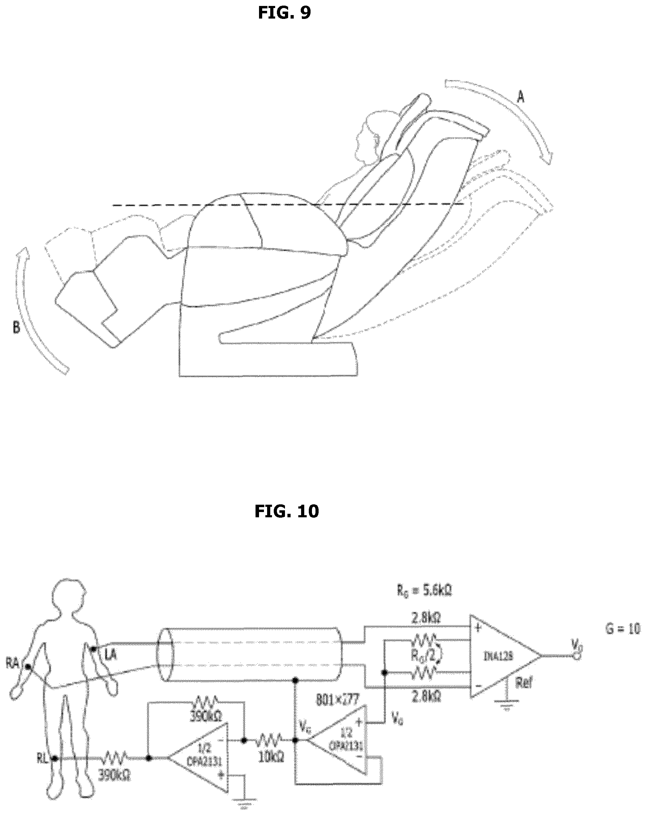

According to an embodiment of the present disclosure, in a case of measuring the body composition of a user, the controller 1200 may adjust a posture of the massage device 1000. For example, the controller 1200 may measure the body composition of a user while the body massage part 100 is reclined. Also, the controller 1200 may measure the body composition of a user in a state in which the leg massage part 300 is lifted. This will be described in detail below with reference to FIG. 9.

According to another embodiment of the present disclosure, the massage device 1000 may utilize at least one of the first body composition measurement module 310 and the second body composition measurement module 1940 to acquire electrocardiograph (ECG) information of the user. For example, the massage device 1000 may use a voltage obtained by the first body composition measurement module 310 as a reference potential and may utilize the second body composition measurement module 1940 to acquire an ECG.

FIG. 10 is a view for describing a concept of a method of utilizing the first body composition measurement module 310 and the second body composition measurement module 1940 to acquire an ECG.

The sensor part 1300 may use at least one sensor to acquire various pieces of information. Examples of the sensor may include a pressure sensor, an infrared sensor, a light emitting diode (LED) sensor, and the like but are not limited thereto.

Also, the sensor part 1300 may include a biometric information acquisition sensor. The biometric information acquisition sensor may acquire fingerprint information, facial information, voice information, iris information, body weight information, electrocardiogram information, body composition information, and the like, but the present disclosure is not limited thereto, and the biometric information acquisition sensor may acquire various other pieces of biometric information.

According to another embodiment of the present disclosure, the massage device 1000 may sense an area in contact with the user and/or a location of the area in contact with the user through sensors. Also, the massage device 1000 may acquire shoulder position information of the user through the sensor part 1300. Also, the massage device 1000 may provide a customized massage on the basis of the acquired information. For example, in the case in which the massage device 1000 provides a shoulder massage, the massage device 1000 may recognize the positions of the user's shoulders on the basis of information acquired through the sensor part 1300 and provide a shoulder massage to the user according to the result of recognition.

The user input part 1800 may receive a command related to operational control of the massage device 1000 from the user, and the user input part 1800 may be implemented in various forms. For example, the user input part 1800 may be disposed in the body massage part 100 or disposed in the leg massage part 300, but the present disclosure is not limited thereto.

The massage device 1000 may acquire various commands from the user through the user input part 1800. For example, the massage device 1000 may receive an arbitrary command relating to selection of massage module, selection of massage type, selection of massage intensity, selection of massage time, selection of massage site, selection relating to location and operation of the body massage part 100, selection relating to on-off of power of the massage device 1000, selection relating to whether to use warming function, selection relating to sound source playback, and the like, but the present disclosure is not limited thereto.

According to another embodiment of the present disclosure, the user input part 1800 may have, according to a function preset by the user, a function preset by itself, or the like, hot key buttons, and/or selection buttons for executing direction selection, cancellation, and input.

The user input part 1800 may be implemented with a key pad, a dome switch, a touch pad (static pressure/capacitive), a jog wheel, a jog switch, and the like, but the present disclosure is not limited thereto. Also, the user input part 1800 may acquire a command through the user's speech on the basis of a voice recognition technology.

According to an embodiment of the present disclosure, the user input part 1800 may include a display configured to display an operational status of the massage device 1000, the current condition of the user, or the like. In this case, the display may be at least one of a liquid crystal display (LCD), a thin film transistor-liquid crystal display (TFT-LCD), an organic light-emitting diode (OLED) display, a flexible display, and a 3D display, but the present disclosure is not limited thereto.

The audio output module 1600 may provide an audio output in an arbitrary form to the user. For example, the audio output module 1600 may output a sound source and/or a binaural beat, which is optimized for a massage pattern provided from the massage device 1000, to the user and provide brain stimulation to the user. The audio output module 1600 may output an acoustic signal which is received through a network (not illustrated) or stored in an internal/external storage medium (not illustrated). For example, through network connection (for example, Bluetooth connection etc.) with a user terminal 2000, the audio output module 1600 may output a sound source according to control of the user terminal 2000. Also, the audio output module 1600 may output an acoustic signal in an arbitrary form that is generated in relation to the operation of the massage device 1000.

The massage device 1000 according to an embodiment of the present disclosure may include the network connection part 1400. The network connection part 1400 may perform communication with a module inside the massage device 1000, an external massage device, and/or the user terminal 2000 through a network in an arbitrary form. The network connection part 1400 may include a wired/wireless connection module for network connection. For example, as a wireless connection technology, wireless LAN (WLAN) (Wi-Fi), wireless broadband (WiBro), World Interoperability for Microwave Access (WiMax), High Speed Downlink Packet Access (HSDPA), and the like may be used. For example, as a wired connection technology, x Digital Subscriber Line (xDSL), Fiber to the Home (FTTH), Power Line Communication (PLC), and the like may be used. Also, the network connection part may include a short-range communication module and transmit and receive data to and from an arbitrary device/terminal located a short distance away. For example, as a short-range communication technology, Bluetooth, Radio Frequency Identification (RFID), Infrared Data Association (IrDA), Ultra Wideband (UWB), ZigBee, and the like may be used, but the present disclosure is not limited thereto.

A storage part 1500 may store various pieces of information relating to the massage device 1000. For example, the storage part 1500 may include massage control information or include personal authentication information, but the present disclosure is not limited thereto.

The storage part 1500 may be implemented through a nonvolatile storage medium that may continuously store arbitrary data. For example, the storage part 1500 may include a disk, an optical disk, and a magneto-optical storage device and also include a flash memory and/or a storage device based on a battery-backup memory, but the present disclosure is not limited thereto.

Also, the storage part 1500 may include a memory. The memory may be a main storage device directly accessed by a processor and may refer to a volatile storage device in which stored information is erased instantaneously when the power is turned off, such as a random access memory (RAM) such as a dynamic random access memory (DRAM) and a static random access memory (SRAM), but the memory is not limited thereto. The memory may be operated by the controller 1200.

FIG. 9 is a view for describing an embodiment in which a posture of the massage device 1000 is adjusted for body composition measurement according to an embodiment of the present disclosure.

According to an embodiment of the present disclosure, the controller 1200 may control the operation of the first body composition measurement module 310 and/or the second body composition measurement module 1940. For example, in a case in which a user has input a command to measure body composition, the controller 1200 may adjust a posture of the massage device 1000 and then perform body composition measurement.

First embodiment of posture adjustment of the massage device 1000:
According to an embodiment of the present disclosure, the massage device 1000 may lift the leg massage part 300 of the massage device 1000 and then perform body composition measurement (B). For example, the massage device 1000 may operate the leg angle actuator provided at the body massage part 100 to lift the leg massage part 300, and the controller 1200 may lift the leg massage part 300 and then perform body composition measurement.

Second embodiment of posture adjustment of the massage device 1000:
According to an embodiment of the present disclosure, the massage device 1000 may recline the body massage part 100 and then perform body composition measurement (A). For example, the massage device 1000 may operate the back angle actuator provided at the body massage part 100 to recline the body massage part 100, and the controller 1200 may recline the body massage part 100 and then perform body composition measurement.

Third embodiment of posture adjustment of the massage device 1000:
According to an embodiment of the present disclosure, the massage device 1000 may lift the leg massage part 300 of the massage device 1000 (B) and may recline the body massage part 100 and then perform body composition measurement (A). For example, the massage device 1000 may operate the leg angle actuator provided at the body massage part 100 to lift the leg massage part 300, operate the back angle actuator to recline the body massage part 100, and then perform body composition measurement.

Those of ordinary skill in the art should well understand that the present disclosure may be implemented by combination of different program modules and/or combination of hardware and software. For example, the present disclosure may be implemented by a computer-readable medium.

Any computer-accessible medium may be the computer-readable medium, and the computer-readable medium includes volatile and nonvolatile media, transitory and non-transitory media, and removable and non-removable media. As a non-limiting example, the computer-readable medium may include a computer-readable storage medium and a computer-readable transmission medium.

The computer-readable storage medium includes volatile and nonvolatile media, transitory and non-transitory media, and removable and non-removable media that may be implemented using computer-readable instructions, a data structure, a program module, or any other arbitrary method or technology for storing information such as data. The computer-readable storage medium may include a RAM, a read-only memory (ROM), an electrically erasable and programmable read-only memory (EEPROM), a flash memory, or other memory technologies, a compact disk-read only memory (CD-ROM), a digital video disk (DVD), or other optical disk storage devices, a magnetic cassette, a magnetic tape, a magnetic disk storage device, or other magnetic storage devices, or any other arbitrary medium that is computer-accessible and may be used to store desired information, but the present disclosure is not limited thereto.

The computer-readable transmission medium generally implements a computer-readable instruction, a data structure, a program module, or any other data in a modulated data signal, such as a carrier wave and any other transport mechanism, and includes any information transmission medium. The term "modulated data signal" refers to a signal in which one or more characteristics of the signal are set or changed to encode information into the signal. As a non-limiting example, the computer-readable transmission medium includes wired media such as a wired network or direct-wired connection and wireless media such as sound, RF, infrared light, and any other wireless medium. Any arbitrary combination of the above-mentioned media also belongs to the range of the computer-readable transmission medium.

Those of ordinary skill in the art should understand that various exemplary logical blocks, modules, processors, means, circuits, and algorithm operations, which have been described in relation to the embodiments disclosed herein, may be implemented by electronic hardware, various forms of programs or design codes (which are referred to as "software" herein for convenience), or any combination thereof. In order to clearly describe the intercompatibility of hardware and software, in relation to functions thereof, various exemplary components, blocks, modules, circuits, and operations have been generally described above. Whether the functions are implemented as hardware or software depends on design constraints imposed on specific applications and the entire system. Those of ordinary skill in the art may implement the described functions using various methods for each specific application, but such implementation decisions should not be interpreted as deviating from the scope of the present disclosure.

Various embodiments proposed herein may be implemented as a manufactured article using a method, a device, or standard programming and/or engineering technology. The term "manufactured article" includes a computer program accessible from an arbitrary computer-readable device, a carrier, or media. For example, the computer-readable storage medium may include a magnetic storage device (e.g., a hard disk, a floppy disk, a magnetic strip, etc.), an optical disk (e.g., a CD, a DVD, etc.), a smart card, and a flash memory device (e.g., an EEPROM, a card, a stick, a key drive, etc.), but is not limited thereto. The term "machine-readable medium" includes wireless channels and various other media capable of storing, retaining, and/or transmitting a command(s) and/or data, but is not limited thereto.

It should be understood that a specific order or hierarchical structure of operations in the proposed processes is an example of exemplary approaches. It should be understood that a specific order or hierarchical structure of operations in the processes may be rearranged within the scope of the present disclosure on the basis of design priorities. The attached method claims provide elements of various operations in a sample order, but this does not mean that the present disclosure is limited to the proposed specific order or hierarchical structure.

Description of the proposed embodiments has been provided above to allow anyone of ordinary skill in the art to use or embody the present disclosure. It should be apparent to those of ordinary skill in the art that various modifications may be made to the embodiments, and general principles defined herein may be applied to other embodiments without departing from the scope of the present disclosure. Therefore, the present disclosure is not limited to the embodiments proposed herein and should be interpreted as having the broadest possible range that is consistent with the principles and novel features proposed herein.

## Claims

1. A massage device comprising:
a massage module configured to provide mechanical stimuli to at least a part of the body of a user;
a first body composition measurement module provided on a leg massage part to measure the body composition of the user; and
a controller configured to control the operation of the massage module and the first body composition measurement module.

2. The massage device of claim 1, wherein the first body composition measurement module includes:
an upper housing which includes an empty space formed therein and at least one opening formed in an upper portion thereof;
a first electrode part which has at least a portion inserted into the upper housing and which protrudes to the outside of the upper housing through the at least one opening;
an elastic part configured to allow the first electrode part and a lower housing to be elastically coupled to each other; and
the lower housing coupled to the upper housing.

3. The massage device of claim 2, wherein a protruding part is provided on a lower portion of the first electrode part, the protruding part passes through a hole provided in the lower housing and protrudes to the outside of the first body composition measurement module, and information on a user is transmitted to the massage device through the protruding part.

4. The massage device of claim 1, further comprising:
an arm massage part configured to massage an arm of the user; and
a second body composition measurement module provided on the arm massage part,
wherein the arm massage part includes an arm massage part frame constituting a framework of the arm massage part and a hand acupressure part configured to apply acupressure to a hand of the user, and
the second body composition measurement module is provided on the hand acupressure part.

5. The massage device of claim 4, wherein, in a case of performing body composition measurement, the controller adjusts a posture of the massage device and then performs the body composition measurement.

6. The massage device of claim 5, wherein the massage device lifts the leg massage part and then performs the body composition measurement

7. The massage device of claim 5, wherein the massage device reclines a body massage part and then performs the body composition measurement.

8. The massage device of claim 5, wherein the massage device lifts the leg massage part, reclines the body massage part, and then performs the body composition measurement.

9. A method of measuring body composition using a massage device including a body massage part and a leg massage part, the method comprising:
receiving an input relating to body composition measurement;
lifting the leg massage part; and
after the leg massage part is lifted, performing the body composition measurement.

10. A method of measuring body composition using a massage device including a body massage part and a leg massage part, the method comprising:
receiving an input relating to body composition measurement;
reclining the body massage part; and,
after the body massage part is reclined, performing the body composition measurement.

11. The method of claim 9, further comprising reclining the body massage part,
wherein, in the performing of the body composition measurement, body composition is measured after the leg massage part is lifted and the body massage part is reclined.
